# EUROPÄISCHE PATENTSCHRIFT

(11) **EP 0 808 297 B1**
(45) Veröffentlichungstag und Bekanntmachung des Hinweises auf die Patenterteilung: **12.05.1999**
(21) Anmeldenummer: 96902925.5
(22) Anmeldetag: 26.01.1996
(51) Int. Cl.: C05G 3/08, C07D 231/12, C07D 231/16

(54) **VERWENDUNG VON SCHWERFLÜCHTIGEN PYRAZOLDERIVATEN MIT HYDROPHILEN GRUPPEN ALS NITRIFIKATIONSINHIBITOREN**
USE OF NOT EASILY VOLATILISED PYRAZOL DERIVATIVES WITH HYDROPHILIC GROUPS AS NITRIFICATION INHIBITORS
UTILISATION DE DERIVES DE PYRAZOLE PEU VOLATILS AVEC DES GROUPES HYDROPHILES COMME INHIBITEURS DE LA NITRIFICATION

(30) Priorität: 06.02.1995 DE 19503827
(43) Veröffentlichungstag der Anmeldung: 26.11.1997
(73) Patentinhaber: BASF AKTIENGESELLSCHAFT, 67056 Ludwigshafen (DE)
(72) Erfinder: RITTINGER, Stefan, D-68199 Mannheim (DE); RIEBER, Norbert, D-68259 Mannheim (DE); GOLD, Randall, Evan, D-67117 Limburgerhof (DE); DRESSEL, Jürgen, D-67141 Neuhofen (DE)
(86) Internationale Anmeldenummer: EP9600319
(87) Internationale Veröffentlichungsnummer: WO9624566

(56) Entgegenhaltungen:
- EP-A- 0 139 183
- EP-A- 0 278 445
- DE-A- 3 409 317
- US-A- 3 303 200
- US-A- 3 635 690
- US-A- 4 084 955
- DATABASE WPI Section Ch, Week 8950 Derwent Publications Ltd., London, GB; Class C04, AN 89-369676 XP002003264 & SU,A,1 488 291 (DZERZ NITROGEN IND) , 23.Juni 1989

## Beschreibung

Die vorliegende Erfindung betrifft die Verwendung von Pyrazolderivaten der Formel I 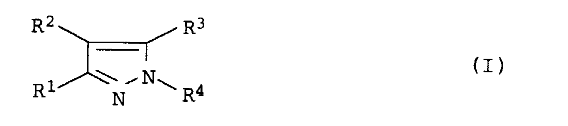 oder deren Säureadditionssalze als Nitrifikationsinhibitoren, in der die Reste R¹, R², R³ und R⁴ die folgende Bedeutung haben:
- R¹, R² und R³ Halogen, Nitro oder Wasserstoff, C₁- bis C₂₀-Alkyl, C₃- bis C₈-Cycloalkyl, C₅- bis C₂₀-Aryl oder Alkylaryl, wobei diese 4 Reste einfach oder zweifach durch Halogen und/oder Hydroxyl substituiert sein können,
- R⁴ ein Rest R^{4a} mit der Formel II 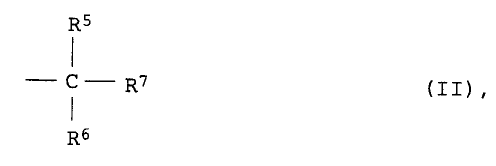 in der
   - R⁵ und R⁶ Wasserstoff, C₁- bis C₂₀-Alkyl, das einfach oder zweifach durch Halogen und/oder Hydroxyl substituiert sein kann, eine Carboxylgruppe, eine Carboxymethylgruppe oder ein funktionelles Derivat der beiden letztgenannten Gruppen ist, und
   - R⁷ ein Carboxylrest oder ein Carboxy-(C₁- bis C₃-alkyl)rest oder ein funktionelles Derivat dieser Gruppen ist.

Weiterhin betrifft die Erfindung neue Pyrazolderivate, die für diesen Verwendungszweck geeignet sind, Verfahren zu deren Herstellung sowie Düngemittelmischungen, die solche Pyrazolderivate enthalten.

Um Pflanzen den von ihnen benötigten Stickstoff zur Verfügung zu stellen, werden sie bekanntlich mit Ammoniumverbindungen gedüngt. Ammoniumverbindungen werden im Boden jedoch in relativ kurzer Zeit mikrobiell zu Nitrat umgesetzt (Nitrifikation).

Weil Nitrat leichter aus dem Boden ausgewaschen wird als Ammonium und der ausgewaschene Anteil für die Pflanzenernährung nicht mehr zur Verfügung steht, ist eine schnelle Nitrifikation jedoch unerwünscht. Damit der Dünger besser ausgenutzt wird, wurde deshalb vorgeschlagen, dem Boden Nitrifikationsinhibitoren zuzusetzen.

Aus der EP-A 474 037 sind 3-Methylpyrazolderivate bekannt, bei denen ein Stickstoffatom des Pyrazolrings einen Methyl-, Cycloalkyl-, Aryl- und Aralkylrest trägt. Es wird vorgeschlagen, diese Verbindungen als Zwischenprodukte zur Herstellung von pharmazeutischen Produkten oder Pflanzenschutzmitteln zu verwenden.

Aus der US-A 3 635 690 ist bekannt, zur Nitrifikationshemmung verschiedene Pyrazolderivate einzusetzen. Bei den vorgeschlagenen Pyrazolderivaten tragen die Stickstoffatome im Pyrazolring keine Substituenten oder eines der Stickstoffatome trägt einen gesättigten oder ungesättigten C₁- bis C₃-Kohlenwasserstoffrest. Ein oder mehrere Kohlenstoffatome im Pyrazolring können durch Kohlenwasserstoffreste, die gegebenenfalls Halogene als Substituenten tragen, substituiert sein. Beispiele hierfür sind 3-Methylpyrazole wie 4-Chlor-3-methylpyrazol.

In der US-A-4 084 955 werden allgemein Di- und Trihalogenpyrazole mit einem Carbonsäurerest in der 1-Position beschrieben.

Die US-A-3 303 200 offenbart Derivate des 1-Hydroxymethylpyrazols, wobei als eine unter vielen möglichen Substituenten am 1-Hydroxyethyl-Rest auch Carbalkoxy zugelassen ist.

Die in der EP-A-278 445 beschriebenen Verbindungen sind Nitrifikationsinhibitoren mit einer geringen Flüchtigkeit.

In der US-A-4 969 946 ist ebenfalls die Verwendung von Pyrazolderivaten als Nitrifikationsinhibitoren beschrieben. Bei diesen Derivaten sind die Stickstoffatome im Pyrazolring unsubstituiert, und mehrere der Ring-Kohlenstoffatome sind durch Halogen oder Methyl substituiert.

Die DE-A 3 704 359 betrifft Mittel, die die Nitrifikation hemmen. Sie enthalten als Wirkstoff Pyrazolderivate, die an einem Stickstoffatom des Pyrazolrings einen Rest der Formel 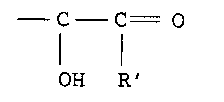 tragen, wobei R' ein substituierter oder unsubstituierter organischer Rest oder ein Hydroxyrest bedeutet.

Weitere Nitrifikationsinhibitoren sind aus der EP-A 0 160 804 bekannt. Es handelt sich um Pyrazolderivate, bei denen ein Stickstoffatom im Pyrazolring einen substituierten Oxycarbonyl-, Sulfinyl-oder Sulfonylrest trägt.

In der DE-A 4 018 395 sind als Nitrifikationsinhibitoren Derivate des 1-Cyanyl-3-methylpyrazols sowie dessen Säureadditionsprodukte bzw. Koordinationsverbindungen genannt.

Aus der SU-A 1 470 737 sind N-Hydroxymethyl-Derivate von Pyrazol bekannt.

Diese Wirkstoffe können als Nitrifikationsinhibitoren noch nicht voll befriedigen, da sie einen oder mehrere der folgenden Nachteile aufweisen:
- zu geringe Wirksamkeit bezogen auf die Auftragsmenge;
- zu geringe Hydrolysestabilität, wodurch die Wirkungsdauer im Boden und die Lagerstabilität herabgesetzt ist. Dies betrifft vor allem Pyrazolderivate wie N-Glyoxylpyrazole und N-Hydroxymethylpyrazole sowie mineralsäure Salze von Pyrazolen;
- zu hohe Toxizität oder Ökotoxizität;
- nur durch aufwendige Herstellungsverfahren zugänglich (z.B. N-Hydroxypyrazole);
- zu geringe Sorption der Verbindungen im Boden, wodurch sie rasch ausgewaschen werden
- zu hohe Flüchtigkeit. Dies hat zur Folge, daß die Verbindungen leicht vom Boden in die Atmosphäre abgegeben werden, es sei denn, die Verbindungen werden mit technisch aufwendigen Verfahren (z.B. per Sonde) in den Boden eingetragen.

Die Aufgabe der vorliegenden Erfindung bestand deshalb darin, Substanzen zu finden bzw. zur Verfügung zu stellen, die als Nitrifikationsinhibitoren wirken und welche die genannten Nachteile nicht aufweisen.

Im einzelnen erstreckte sich die Aufgabe auf das Auffinden bekannter Verbindungen mit den gewünschten Eigenschaften sowie der Synthese neuer Verbindungen mit derartigen Eigenschaften.

Demgemäß wurde gefunden, daß sich die Pyrazolderivate der Formel I als Nitrifikationsinhibitoren eignen. Weiterhin wurden die erfindungsgemäßen Verbindungen der Formel Ia 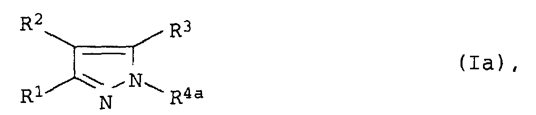 in der
- R¹ und R³ die für die Formel I angegebene Bedeutung haben,
- R² Halogen und
- R⁴ ein Rest mit der Formel II ist, wobei die Substituenten R⁵, R⁶ und R⁷ die für die Formel I angegebene Bedeutung haben,
bedeutet, sowie die Säureadditionssalze der Verbindungen der Formel Ia bereitgestellt.

Für die Verwendung als Nitrifikationsinhibitoren eignen sich insbesondere solche Verbindungen, bei denen die Substituenten R¹, R² und R³ C₁- bis C₅-Alkyl oder Halogen bedeuten. Als Alkylreste kommen vor allem Methyl und Ethyl und als Halogenreste Br, Cl und F in Betracht. Besonders bevorzugt enthalten die Pyrazolderivate der Formel I Einheiten, die von 4-Methylpyrazol, 4-Chlorpyrazol, 3-Methylpyrazol, 5-Methylpyrazol, 4-Chlor-3-Methylpyrazol und 4-Chlor-5-Methylpyrazol 3,4-Dimethylpyrazol abgeleitet sind.

Als Reste R⁴ kommen Reste R^{4a} mit der Formel II 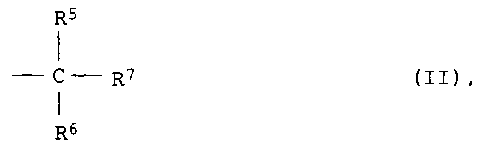 in der
- R⁵ und R⁶ Wasserstoff, C₁- bis C₂₀-Alkyl, das einfach oder zweifach durch Halogen und/oder Hydroxyl substituiert sein kann, eine Carboxylgruppe, eine Carboxymethylgruppe oder ein funktionelles Derivat der beiden letztgenannten Gruppen ist und
- R⁷ ein Carboxylrest oder ein Carboxy- C₁- bis C₃-alkylrest oder ein funktionelles Derivat dieser Reste ist,
in Frage.

Unter den Resten R⁵ und R⁶ in der Formel II sind folgende Gruppen bevorzugt:
Unsubstituierte Alkylreste, insbesondere C₁- bis C₆-Alkylreste wie Methyl, Ethyl und Propyl sowie Wasserstoff. Daneben kommen vor allem Carboxymethylgruppe oder funktionelle Gruppen der Carboxymethylgruppen in Betracht.

Bevorzugte Reste R⁵, R⁶ und R⁷, die ein funktionelles Derivat einer Carboxylgruppe enthalten, sind solche, bei denen die Carboxylgruppe durch eine Gruppe ersetzt ist, die zu einer Carboxylgruppe hydrolysiert werden kann, also beispielsweise durch C₁- bis C₆-Alkoxycarbonyl oder gegebenenfalls mit C₁- bis C₆-Alkylgruppen mono- oder disubstituiertes Carbamoyl. Selbstverständlich kann die Carboxylgruppe auch in neutralisierter Form vorliegen, d.h. in Salzform, beispielsweise in Form eines Ammonium- oder Alkalisalzes.

Zu den funktionellen Derivaten der Carboxylgruppe zählt auch die Carbonyldioxygruppe über die jeweils 2 der R⁵, R⁶ und R⁷ verbunden sein können.

Besonders bevorzugt sind solche Reste R^{4a}, in denen R⁵ Wasserstoff und R⁶ Carboxyl, Carboxymethyl, Methyl oder ebenfalls Wasserstoff ist.

Der definitionsgemäße Rest R⁷ ist oder enthält bevorzugt eine Carboxylgruppe. Die Carboxylgruppe kann auch durch ein funktionelles Derivat der Carboxylgruppe ersetzt sein, wobei die Gruppen bevorzugt sind, die auch für die Reste R⁵ und R⁶ in Betracht kommen. Ganz besonders bevorzugt sind Pyrazolderivate, die die folgenden Reste tragen:
- R⁵ Wasserstoff
- R⁶ Wasserstoff, Methyl, eine Carboxylgruppe, eine Carboxymethylgruppe oder ein funktionelles Derivat der beiden letztgenannten Gruppen
- R⁷ eine Carboxymethylgruppe, eine 1-Carboxyethylgruppe oder funktionelle Derivate dieser Gruppen.
Dabei sind folgende Kombinationen bevorzugt:
- R⁵ Wasserstoff, R⁶ Carboxyl, R⁷ Carboxymethyl
- R⁵ Wasserstoff, R⁶ Wasserstoff, R⁷ Carboxymethyl
- R⁵ Wasserstoff, R⁶ Wasserstoff R⁷ 1-Carboxyethyl

Die Pyrazolderivate der Formel I, bzw. Ia, die einen Rest R4a tragen, lassen sich auf einfache Weise herstellen, indem man ein Pyrazolderivat der Formel V 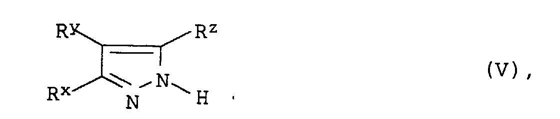 in der
I.
   - R^{x} die gleiche Bedeutung wie R¹
   - R^{y} die gleiche Bedeutung wie R²
   - R^{z} die gleiche Bedeutung wie R³
   oder
II.
   - R^{x} die gleiche Bedeutung wie R³
   - R^{y} die gleiche Bedeutung wie R²
   - R^{z} die gleiche Bedeutung wie R¹
   hat bzw. Va 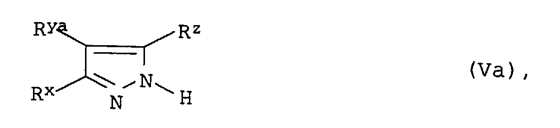 in der R^{ya} Halogen bedeutet
   mit einer Verbindung der Formel VI 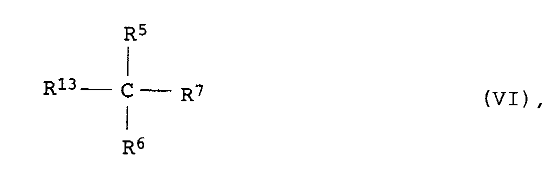 in der der Rest R¹³ Chlor oder Brom ist umsetzt.

Bei den Säureadditionssalzen der Verbindungen der Formeln I, Ia und Ib handelt es sich bevorzugt um solche, die sich von Mineralsäuren, z.B. Salzsäure, Schwefelsäure und Phosphorsäure ableiten.

Günstigerweise nimmt man die Reaktion bei Temperaturen von 0 bis 150°C unter Normaldruck in Abwesenheit eines Lösungsmittels oder in einem inerten Lösungsmittel, z.B. Acetonitril oder Dimethylsulfoxid, in Gegenwart von, bezogen auf das Ausgangsprodukt der Formel III, in etwa äquimolaren Mengen eines Alkalihydroxids wie Natriumhydroxid und katalytischen Mengen eines Phasentransferkatalysators vor. Als Phasentransferkatalysatoren eignen sich C₁₀- bis C₃₀-Alkylammoniumhydroxide, z.B. Octyltriethylammoniumhydroxid.

Nach einem anderen Verfahren erhält man die Pyrazolderivate der Formel I bzw. Ia, die einen Rest R^{4a} tragen, indem man ein Pyrazolderivat der Formel V bzw. Va in einer Additionsreaktion mit einer Verbindung der Formel VII 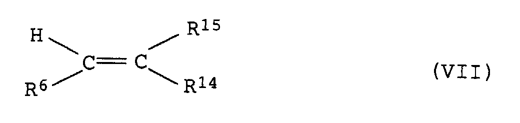 umsetzt, in der
- der Rest R⁶ Wasserstoff oder eine Carboxylgruppe oder ein funktionelles Derivat einer Carboxylgruppe ist,
- der Rest R¹⁴ eine Carboxylgruppe oder ein funktionelles Derivat einer Carboxylgruppe und
- der Rest R¹⁵ Wasserstoff oder Methyl ist.

Als Rest R¹⁴ kommen insbesondere solche funktionellen Derivate der Carboxylgruppe in Betracht, durch die auch die Carboxylgruppe im Rest R⁶ ersetzt sein kann.

Besonders einfach läßt sich die Umsetzung mit Maleinsäureanhydrid, Acrylsäure und Methacrylsäure sowie den Alkylestern der beiden genannten Säuren durchführen.

Für die Reaktionsbedingungen Druck und Temperatur gilt das für die Umsetzung von Verbindungen der Formel V mit denen der Formel VI gesagte.

Als Lösungsmittel können neben denen, die für die Umsetzung von Verbindungen der Formel V mit denen der Formel VI geeignet sind, auch allgemein übliche Lösungsmittel, also Alkohole, Ether, Ketone, Wasser sowie Alkane eingesetzt werden.

Die Pyrazolderivate I bzw. Ib, die einen Rest R^{4b} tragen, lassen sich auf einfache Weise herstellen, indem man ein Pyrazolderivat der Formel V bzw. Va mit einem entsprechenden Epoxid der Formel VIII 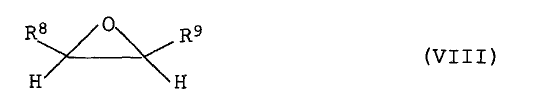 das die gewünschten Reste R⁸ und R⁹ trägt, umsetzt. Diese Additionsreaktion ist allgemein bekannt und kann nach den allgemein bekannten Methoden (vgl. Methoden der Organischen Chemie (Houben-Weyl), 4. Auflage 1979, Band VI/1a, Alkohole I, Teil 1, S. 373 bis 380) erfolgen. Dabei bestimmt die Menge des eingesetzten Epoxids VII die Größe von X.

Die bevorzugten Pyrazolderivate, die mehrere Ethylenoxid- und/oder Propylenoxid-Einheiten enthalten, lassen sich besonders günstig nach einem der Verfahren herstellen, die in Methoden der Organischen Chemie (Houben-Weyl), 4. Auflage 1979, Band XIV/2, Makromolekulare Stoffe, Teil 2, S. 425 bis 460 beschrieben ist. Im allgemeinen wird die Umsetzung bei Temperaturen von 40 bis 200, bevorzugt 80 bis 140°C unter einem Überdruck durchgeführt. Der Druck ist bevorzugt derjenige, der sich bei der gewünschten Reaktionstemperatur in einem verschlossenen Reaktionsgefäß von selbst einstellt, wenn das Volumen des Reaktionsgefäßes so gewählt wird, daß die Ausgangsstoffe in flüssiger oder fester Form mindestens 20, bevorzugt mindestens 50 % seines Volumens ausfüllen.

Die Reaktion läuft unter den genannten Bedingungen zwar auch ohne Zusatz eines Katalysators ab, es empfiehlt sich jedoch, den Ausgangsstoffen zur Beschleunigung der Reaktion katalytische Mengen Wasser, Alkalihydroxiden wie Natriumhydroxid oder anorganischen Säuren wie Schwefelsaure oder eines Aktivators wie Aluminiumoxid zuzusetzen.

Bei der Auswahl eines Pyrazolderivates der Formel V bzw. Va ist zu berücksichtigen, daß ein Rest R^{x} in einem Pyrazolderivat der Formel V bzw. Va in einem Pyrazolderivat der Formel I einem Rest R¹, ein Rest R^{y} einem Rest R² und ein Rest R^{z} einem Rest R³ entspricht.

Weil die Pyrazolderivate der Formel III unter den Reaktionsbedingungen, in denen die Reaktion üblicherweise durchgeführt wird, tautomerisieren 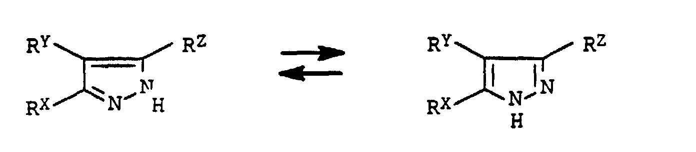 läßt es sich im allgemeinen nicht vermeiden, daß, falls die Substituente R^{x} und RY, verschieden sind, Isomerengemische der Pyrazolderivate der Formel I gebildet werden, die auch ein Strukturisomeres enthalten, in welchem dem Rest R¹ ein Rest R^{z} aus dem Pyrazolderivat der Formel III und dem Rest R³ ein Rest R^{x} entspricht.

Die Säureadditionssalze der Verbindungen der Formeln I, Ia und Ib sind durch Umsetzung dieser Verbindungen mit den entsprechenden Säuren erhältlich, z.B. indem man Lösungen dieser Verbindungen mit stöchiometrischen Mengen von Säuren versetzt und das Lösungsmittel anschließend entfernt.

Die Pyrazolderivate sind sowohl in fester Form als auch in Form ihrer konzentrierten wässerigen Lösungen handelsfahig.

Die Anwendung der Pyrazolderivate der Formel I als Nitrifikationsinhibitoren erfolgt nach den allgemein Üblichen Verfahren: Sie können also beispielsweise in fester Form als Pulver oder Granulat direkt auf den Boden aufgebracht werden.

Es hat sich auch bewährt, Mischungen des Nitrifikationsinhibitors mit einem Mineraldünger, insbesondere einem Stickstoff-Dünger zuzusetzen. Solche Düngemittelmischungen enthalten bevorzugt 0,01 bis 1 Gew.-% an Nitrifikationsinhibitor, bezogen auf den Mineraldünger.

Die Pyrazolderivate mit Carboxylgruppen oder 2 bis 6 Ethylenoxid- oder Propylenoxid-Einheiten sind besonders bevorzugt, weil sie gut wasserlöslich sind. Die Wasserlöslichkeit ist deshalb erwünscht, weil sich wässerige Lösungen der Nitrifikationsinhibitoren beim Auftrag auf den Boden besonders gut dosieren lassen und sich mit ihnen auf einfache Weise hochwirksame Langzeitdünger herstellen lassen.

Insbesondere kommen Düngemittelmischungen, die 20 bis 1000 Gew.-%, bezogen auf die Düngemittelmischung, an Wasser enthalten, in Betracht. Die Wassermenge wird dabei bevorzugt so gewählt, daß die Düngemittelmischung als wässerige Lösung vorliegt.

Besonders bewährt haben sich wegen ihrer guten Langzeitwirkung Düngemittelmischungen, die nach folgender Methode hergestellt werden:
Granulate von Mineraldüngern wie beispielsweise ammoniumhaltige Nitrate, Sulfate oder Phosphate, werden mit einem Nitrifikationsinhibitor imprägniert oder beschichtet, indem man sie mit einer Lösung des Nitrifikationsinhibitors besprüht und wieder trocknet. Die Methode ist beispielsweise aus DE-A 4 128 828 bekannt, auf die hier in vollem Umfang Bezug genommen wird. Die dort zusatzlich vorgeschlagene Versiegelung des imprägnierten Granulats mit einem Paraffinwachs erübrigt sich im allgemeinen auf Grund der wesentlich geringeren Flüchtigkeit der erfindungsgemäßen Nitrifikationsinhibitoren.

Üblicherweise werden die Nitrifikationsinhibitoren in Mengen von 100 g/ha bis 10 kg/ha auf den Boden aufgebracht.

Die auf einfache Weise aus preisgünstigen Ausgangsprodukten herstellbaren Pyrazolderivate der Formel I zeichnen sich bei ihrer Verwendung als Nitrifikationsinhibitoren vor allem dadurch aus, daß sie die Nitrifikation von Ammonium-Stickstoff im Boden über einen langen Zeitraum hinweg wirksam hemmen.

Hinzu kommt, daß diese Verbindungen kaum toxisch sind, einen niedrigen Dampfdruck aufweisen und im Boden gut sorbiert werden. Dies hat zur Folge, daß sie weder in nennenswertem Umfang durch Sublimation in die Atmosphäre abgegeben noch durch Wasser leicht ausgewaschen werden. Hierdurch ergeben sich zum einen ökonomische Vorteile wie eine hohe Wirtschaftlichkeit auf Grund der länger anhaltenden Wirkung der Nitrifikationsinhibitoren und zum anderen ökologische Vorteile wie eine Verringerung der Belastung von Luft und Oberflächengewässern und Grundwasser.

### A. Herstellungsbeispiele

### Beispiel 1 (315932)

8,2 g 3-Methylpyrazol (0,1 mol) und 9,8 g Maleinsäureanhydrid (0,1 mol) wurden in 50 ml 50 %iger Essigsäure auf 100°C erhitzt. Nach 16 h wurde zur Trockene eingedampft. Beim Aufnehmen des Rückstands in Diethylether fällt das Produkt (2-(N-3-Methylpyrazol)bernsteinsäure) rein aus und wird abfiltriert: weiße Kristalle vom Smp. 186°C. Ausbeute 18,2 g (92 %). Im NMR-Spektrum sind 2 Methylsignale (Isomerenverteilung 2:1) erkennbar, was mit der Aufhebung der 3,5-Tautomerie durch die Substitution am Stickstoff im Einklang steht.

### Beispiel 2 (321300)

Eine Lösung aus 10 g 2-(N-3-Methylpyrazolyl)bernsteinsäure in 20 ml Wasser wurde nach Neutralisation mit 4 g NaOH mit 65 ml einer 6 gew.-%igen NaOCl-Lösung versetzt und 12 h bei Raumtemperatur umgesetzt. Danach wurde die Lösung mit Salzsäure auf einen pH-Wert von 2 bis 4 eingestellt und der dabei gebildete Niederschlag abfiltriert. Das Filtrat wurde bis pH 1 angesäuert, zur Trockene eingedampft und in MeOH aufgenommen. Aus der methanolischen Lösung läßt sich durch Neutralisation mit Triethylamin erneut ein Niederschlag erhalten, welcher sich als identisch zum ersten ersten erweist, (IR): weiße Kristalle vom Smp. > 250°C, die NMR-spektroskopischen Daten und MS stehen mit der Struktur von 2-(N-3-Methyl)-4-chlorpyrazolyl)bernsteinsäure im Einklang.

### Beispiel 3 (315573)

6,8 g Pyrazol wurden in 50 ml Acetonitril vorgelegt und mit 5,6 g KOH und 100 mg Octyltrimethylammoniumhydroxid versetzt. Zu dieser Suspension wird bei Raumtemperatur Chloressigsäuremethylester getropft. Nach 12h bei Raumtemperatur hatte sich quantitativ der N-3-Methylpyrazolylessigester gebildet.

Nach Eindampfen zur Trockene wurde der Rückstand in Ether aufgenommen und filtriert. Das Filtrat wurde nach Abdestillieren des Ethers einer Umesterung mit Ameisensäure unterworfen, wonach die flüchtigen Bestandteile mit einem Siedepunkt bis 100°C bei Normaldruck abdestilliert wurden. Der Rückstand wurde aus Toluol umkristallisiert Die gebildete Pyrazolylessigsäure (Ausbeute 89 %) wies einen Smp. von 172°C auf.

### Beispiel 4 (319782)

8,2 g 3-Methylpyrazol wurden wie in Beispiel 3 verarbeitet. Man erhielt 12,7 g (91,5 % d.Th.) N-(3-Methylpyrazolyl)essigsäure als weiße Kristalle vom Smp. 143°C.

### Beispiel 5 (319225)

5,8g (0,05mol) 4-Chlor-3-Methylpyrazol wurden wie in Beispiel 3 verarbeitet. Man erhielt 7,6 g (87 % d.Th.) N-(3-Methyl-4-chlorpyrazolyl)essigsäure als weißes Pulver vom Smp. 155°C.

### Beispiel 6 (321233)

5,1 g 4-Chlorpyrazol wurden wie in Beispiel 3 beschrieben verarbeitet. Es wurden 6,8 g N-(4-Chlorpyrazolyl)essigsäure (Smp. 161 bis 163°C) erhalten.

### Beispiel 7 (176238)

6,8g Pyrazol wurden wie in Beispiel 3 mit 3-Chlorpropionsaureethylester umgesetzt und analog weiterverarbeitet. Man erhält 11,8 g d.Th.) 3-(N-Pyrazolyl)propionsäure als weißes Pulver vom Smp. 70°C.

### Beispiel 8 (319783)

8,2g 3-Methylpyrazol wurden wie in Beispiel 7 verarbeitet. Man erhält 13,8 g 3-(N-3-Methylpyrazolyl)propionsäure als weißes Pulver vom Smp. 95°C.

### Beispiel 9 (319227)

5,8 g (0,05mol)3-Metbyl-4-chlorpyrazol wurden wie in Beispiel 7 verarbeitet. Man erhielt 8,4 g 3-(N-3-methyl-4-chlorpyrazolyl)propionsäure als weißes Pulver. Durch Kristallisation aus Ether wurde eine Modifikation vom Smp.103°C, durch Kristallisation aus Toluol eine Modifikation vom Smp. 66 bis 80°C erhalten.

### Beispiel 10 (321233)

In 82 g 3-Methylpyrazol wurden 50 g Methylmethacrylat (MMA) gelöst und über Nacht bei 100°C gerührt. Nach Fraktionierung im Vakuum erhielt man das ölige Gemisch der 1,3- und 1,5-isomeren Pyrazolderivate, d.h. des 2-Methyl-3-(N-3-methylpyrazolyl)propionmethylesters und des 2-Methyl-3-(N-5-methylpyrazolyl)propionsäuremethylesters (Siedepunkt 82°C (0,01 mbar)). Ausbeute 90 g (99 % bez. auf MMA).

### Beispiel 10a

116,6 g (1,0 mol) 4-chlor-3-methylpyrazol wurden auf 100°C erhitzt und unter Rühren 137 g (1,07 mol) Acrylsäure-n-butylester zugetropft. Nach Rühren über Nacht wurde das Gemisch der isomeren Ester [3-(N-4-Chlor-3-methylpyrazolyl)propionsäure-n-butylester und 3-(N-4-Chlor-5-methylpyrazolyl)propionsäure-n-butylester] im Vakuum fraktioniert (Siedepunkt: 116°C (0,6 mbar)).
Ausbeute: 240 g (98 %).

### Beispiel 10b

116,6 g (1,0 mol) 4-Chlor-3-methylpyrazol wurden mit 107,1 g (1,07 mol) Acrylsäureethylester, wie in Beispiel 10a beschrieben, umgesetzt. Es wurden Produkte erhalten, die zu denen des Beispiels 10a analog waren.

### Beispiel 10c

96,2 g (1,0 mol) 3,4-Dimethylpyrazol wurden, wie in Beispiel 10a beschrieben, umgesetzt. Es wurden Produkte erhalten, die zu denen des Beispiels 10a analog waren.

### Beispiel 11

Zu einer Suspension aus 100 ml Chloroform, 50 g Aluminiumoxid (Aluminiumoxid der Fa. Merck, Aktivitätsstufe 1, Korngröße 0,063 bis 0,2 mm) und 8,2 g (0,1 mol) 3-Methylpyrazol wurde innerhalb von ca. 3 h 4,4 g (0,1 mol) Ethylenoxid, das unterhalb des Siedepunktes abgekühlt worden war, zugetropft. Nach einer Verweilzeit von 12 h bei Raumtemperatur wurde das Aluminiumoxid abfiltriert und 3 mol mit einer Lösung aus 20 Teilen Methanol und 1 Teil Triethylamin gewaschen. Das Filtrat und die Waschlösung wurden vereinigt und die Lösungsmittel entfernt. Es verblieben 12,1 g eines Öls, das zu 95 Gew.-% aus 2-(N-Methylpyrazolyl)ethanol besteht (Isomerenverteilung der 1,3- und 1,5-stellungsisomeren Pyrazolderivate = 2:1).

### Beispiel 12 (319899)

Ein 5 ml Autoklav, in dem 2 g (0,025 mol) 3-Methylpyrazol und 0,1 ml H₂O vorgelegt worden waren, wurde unter Inertgasatmosphäre und bei -20°C mit 2,2 g (0,05 mol) Ethylenoxid befüllt und verschlossen. Der Autoklav wurde anschließend für ca. 12 h auf 120°C erhitzt. Der Reaktionsaustrag bestand neben geringen Mengen an unumgesetztem 3-Methylpyrazol aus Addukten von Ethylenoxid an 3-Methylpyrazol. Diese Addukte sind Mischungen von Pyrazolylderivaten, die aus einer 3-Methylpyrazolyl- bzw. 5-Methylpyrazolyl-Einheiten aufgebaut sind und eine Hydroxyethyl-gruppe oder einen aus mehreren Polyethylenoxid-Einheiten aufgebauten Rest tragen. Der Hauptanteil sind Pyrazolderivate mit einem aus 2 Polyethylenoxid-Einheiten bestehenden Rest (Ausbeute 60 %).

### Beispiel 13 (319306)

Einer Lösung aus 6,8 g (0,1 mol) Pyrazol, 0,1 ml HBF₄ (40 %ige wässerige Lösung) und 50 ml Wasser wurde bei 60°C langsam 5,8 g (0,1 mol) Propylenoxid zugetropft und anschließend 3h bei dieser Temperatur umgesetzt. Die Reaktionsmischung wurde durch fraktionierte Vakuumdestillation aufgearbeitet. Es wurden 10 g einer Fraktion mit einem Siedepunkt von 51°C (0,02 mbar) erhalten. Dabei handelte es sich um ein Isomerengemisch aus 85 % des Monoadduktes von Pyrazol an das C-Atom von Propylenoxid in der 1-Position und 15 % des entsprechenden Adduktes in der 2-Position.

### Beispiel 14 (310226)

11,6 g 4-Cl-3-methylpyrazol (0,1 mol) wurden wie in Beispiel 13 behandelt. Ausbeute 14,2 g (82 %). Sdp. 75°C (0,015 mbar). Im Produktgemisch ist mittels NMR zusätzlich zur in Beispiel 13 beschriebenen Isomerie der Verknüpfung des Heterocyclus mit PO noch die in Beispiel 11 beschriebene 1,3- bzw. 1,5-Isomerie am Ring beobachtbar. Im Gaschromatogramm wurden insgesamt vier Isomere aufgetrennt.

### Beispiel 15 (321234)

2 g (0,025 mol) 4-Methylpyrazol wurden wie in Beispiel 12 beschrieben umgesetzt. Es wurden Produkte erhalten, die zu denen des Beispiels 12 analog waren.

### Beispiel 16 (321235)

2 g (0,025 mol) 4-Chlor-3-Methylpyrazol wurden wie in Beispiel 12 beschrieben umgesetzt. Es wurden Produkte erhalten, die zu denen des Beispiels 12 analog waren.

### Beispiel 17 (321236)

2 g (0,025 mol) 4-Chlorpyrazol wurden wie in Beispiel 12 beschrieben umgesetzt. Es wurden Produkte erhalten, die zu denen des Beispiels 12 analog waren.

### Beispiel 18

8,2 g (0,1 mol) 3-Methylpyrazol und 7,2 g Acrylsäure wurden in 100 ml Tetrahydrofuran (THF) über 16 h im Rückfluß erhitzt. In GC konnte der quantitative Umsatz zu 319783 (Bsp. 8) verfolgt werden. Nach Abziehen des THF im Vakuum wurden durch Umkristallisation aus Toluol 14 g Reinsubstanz (identisch zu Bsp. 8) erhalten.

### Beispiel 18a

116,6 g (1,0 mol) 4-Chlor-3-methylpyrazol wurden auf 100°C erhitzt und unter Rühren 77,1 g (1,07 mol) Acrylsaure zugetropft. Nach Rühren über Nacht wurde eine viskose Flüssigkeit erhalten, die beim Stehen kristallisierte. Nach Umkristallisation aus Aceton erhielt man 113 g (60 %) des Gemisches der isomeren Pyrazolderivate 3-(N-4-Chlor-3-methyl-pyrazolyl)propionsäure und 3-(N-4-Chlor-5-methylpyrazolyl)propionsäure.

### Beispiel 18b

96,2 g (1,0 mol)3,4-Dimethylpyrazol wurden, wie in Beispiel 18a beschrieben, umgesetzt. Es wurden Produkte erhalten, die zu denen des Beispiels 18a analog waren.

### Beispiel 19

11,7 g 4-Chlor-3-Methylpyrazol (0,1 mol) und 7,2 g Acrylsäure (0,1 mol) wurden wie in Bsp. 18 behandelt. Nach Abkühlen der Reaktionslösung wurden durch Zugabe eines zweifachen Überschusses (Vol.) von Penten bei 0°C 18 g Produkt gefällt (weißes Pulver), welches sich spektroskopisch (GC, NMR) als identisch zu Beispiel 9 erwies (319227).

### B. Anwendungstechnische Prüfungen

Flüchtigkeit bei Raumtemperatur

| Beispiele | Dampfdruck [mbar] |
|---|---|
| Beispiel 1 | 1,3 x 10⁻¹³ |
| Beispiel 4 | 1,1 x 10⁻⁵ |
| Beispiel 5 | 2,8 x 10⁻⁷ |
| Beispiel 8 | 1,3 x 10⁻⁵ |
| Beispiel 12 | 5,5 x 10⁻⁴ |

| Vergleichsbeispiele | Dampfdruck [mbar] |
|---|---|
| 3-Methylpyrazol | 7,0 x 10⁻² |
| 4-Chlor-3-methylpyrazol | 2,8 x 10⁻³ |

Die Messung des Dampfdrucks erfolgte bei 20°C gemäß OECD-Richtlinie 79/831/EWG V, aktualisierte Fassung vom Februar 1990, Teil A4.

## Patentansprüche

1. Verwendung von Pyrazolderivaten der allgemeinen Formel I 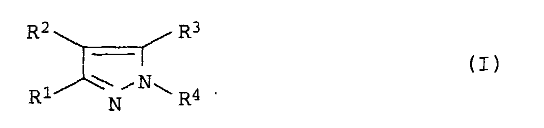 oder deren Säureadditionssalze als Nitrifikationsinhibitoren, in der die Reste R¹, R², R³ und R⁴ die folgende Bedeutung haben:
- R¹, R² und R³ Halogen, Nitro, Wasserstoff, C₁- bis C₂₀-Alkyl, C₃- bis C₈-Cycloalkyl, C₅- bis C₂₀-Aryl oder Alkylaryl, wobei diese 4 Reste einfach oder zweifach durch Halogen und/oder Hydroxyl substituiert sein können
- R⁴ ein Rest R^{4a} mit der Formel II 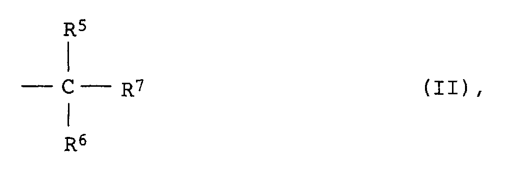
in der
- R⁵ und R⁶ Wasserstoff, C₁- bis C₂₀-Alkyl, das einfach oder zweifach durch Halogen und/oder Hydroxyl substituiert sein kann, eine Carboxylgruppe, eine Carboxymethylgruppe oder ein funktionelles Derivat der beiden letztgenannten Gruppen ist, und
- R⁷ ein Carboxylrest oder ein Carboxy-(C₁- bis C₃-alkyl)rest oder ein funktionelles Derivat dieser Gruppen ist.

2. Verwendung von solchen Pyrazolderivaten nach Anspruch 1 als Nitrifikationshemmer, bei denen die Reste R¹, R² und R³ C₁- bis C₅-Alkyl oder Halogen bedeuten.

3. Pyrazolderivate der Formel Ia 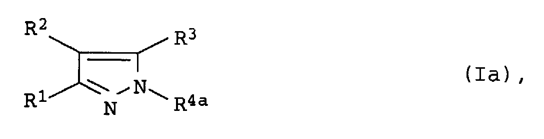 bei denen der Rest R² Halogen ist oder deren Säureadditionssalze, wobei solche Verbindungen ausgenommen sind, bei denen 2 der Reste R¹, R² oder R³ gleichzeitig Halogen bedeuten.

4. Pyrazolderivate nach Anspruch 3, bei denen der Rest R^{4a} als Substituenten die folgenden Gruppen trägt:
- R⁵ Wasserstoff
- R⁶ Wasserstoff oder eine Carboxylgruppe oder ein funktionelles Derivat der Carboxylgruppe
- R⁷ eine Carboxymethylgruppe, eine 1-Carboxyethylgruppe oder ein funktionelles Derivat dieser Gruppen.

5. Verfahren zur Herstellung von Pyrazolderivaten gemäß Anspruch 1, dadurch gekennzeichnet, daß man ein Pyrazolderivat der Formel Va 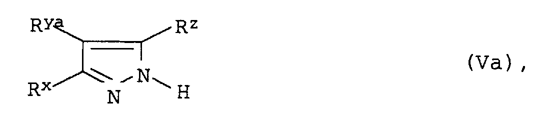 in der
- R^{x} und R^{z} Halogen, Nitro, Wasserstoff, C₁- bis C₂₀-Alkyl, C₃- bis C₈-Cycloalkyl, C₅- bis C₂₀-Aryl oder Alkylaryl, wobei diese 4 Reste einfach oder zweifach durch Halogen und/oder Hydroxyl substituiert sein können, bedeuten und
- R^{ya} Halogen bedeutet
mit einer Verbindung der Formel VI 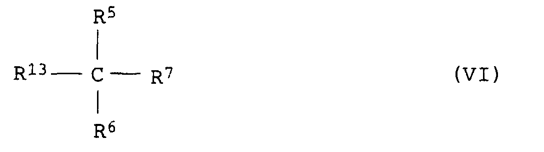 umsetzt, in der R¹³ Chlor oder Brom ist, wobei solche Verbindungen ausgenommen sind, bei denen 2 der Reste R¹, R² oder R³ gleichzeitig Halogen bedeuten.

6. Verfahren zur Herstellung von Pyrazolderivaten gemäß Anspruch 4, dadurch gekennzeichnet, daß man ein Pyrazolderivat der Formel Va mit einer Verbindung der Formel VII 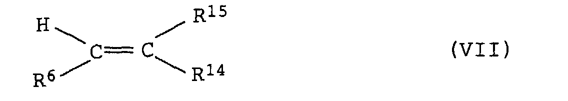 umsetzt, in der
- der Rest R⁶ Wasserstoff oder eine Carboxylgruppe oder ein funktionelles Derivat einer Carboxylgruppe ist,
- der Rest R¹⁴ eine Carboxylgruppe oder ein funktionelles Derivat einer Carboxylgruppe und
- der Rest R¹⁵ Wasserstoff oder Methyl ist.

7. Düngemittelmischungen enthaltend
A. einen Mineraldünger und
B. 100 bis 10000 Gew.-ppm, bezogen auf den Mineraldünger eines Pyrazolderivates nach den Ansprüchen 1 bis 6.

## Claims

1. The use of pyrazole derivatives of the general formula I 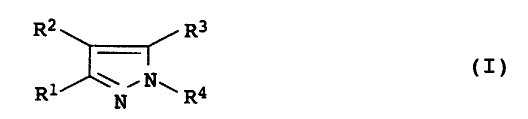 or acid addition salts thereof as nitrification inhibitors, where the radicals R¹, R², R³ and R⁴ have the following meanings:
- R¹, R² and R³ are halogen, nitro, hydrogen, C₁- to C₂₀-alkyl, C₃- to C₈-cycloalkyl, C₅- to C₂₀-aryl or alkylaryl, it being possible for these 4 radicals to be monosubstituted or disubstituted by halogen and/or hydroxyl
- R⁴ is a radical R^{4a} having the formula II 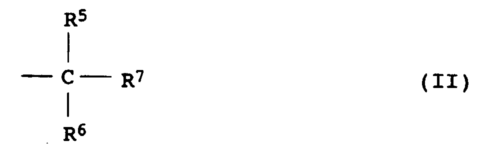 where
- R⁵ and R⁶ are hydrogen, C₁- to C₂₀-alkyl which can be monosubstituted or disubstituted by halogen and/or hydroxyl, a carboxyl group, a carboxymethyl group or a functional derivative of the two last-mentioned groups, and
- R⁷ is a carboxyl radical or a carboxy-(C₁- to C₃-alkyl) radical or a functional derivative of these groups.

2. The use of those pyrazole derivatives as set forth in claim 1 as nitrification inhibitors, where the radicals R¹, R² and R³ are C₁- to C₅-alkyl or halogen.

3. A pyrazole derivative of the formula Ia 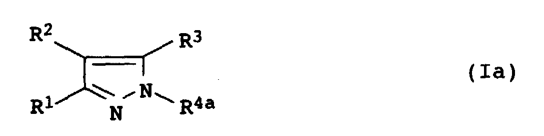 where the radical R² is halogen or an acid addition salt thereof, those compounds being excluded where 2 of the radicals R¹, R² and R³ are simultaneously halogen.

4. A pyrazole derivative as claimed in claim 3, where the radical R^{4a} carries, as a substituent, the following groups:
R⁵ - hydrogen
R⁶ - hydrogen or a carboxyl group or a functional derivative of the carboxyl group
R⁷ - a carboxymethyl group, a 1-carboxyethyl group or a functional derivative of these groups.

5. A process for preparing pyrazole derivatives as set forth in claim 1, which comprises reacting a pyrazole derivative of the formula Va 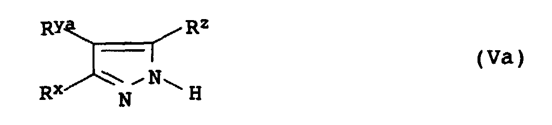 where
- R^{x} and R^{z} are halogen, nitro, hydrogen, C₁- to C₂₀-alkyl, C₃- to C₈-cycloalkyl, C₅- to C₂₀-aryl or alkylaryl, it being possible for these 4 radicals to be monosubstituted or disubstituted by halogen and/or hydroxyl, and
- R^{ya} is halogen
with a compound of the formula VI 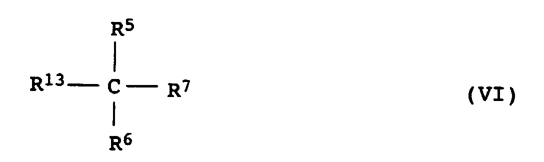 where R¹³ is chlorine or bromine, those compounds being excluded where 2 of the radicals R¹, R² and R³ are simultaneously halogen.

6. A process for preparing pyrazole derivatives as claimed in claim 4, wherein a pyrazole derivative of the formula Va is reacted with a compound of the formula VII 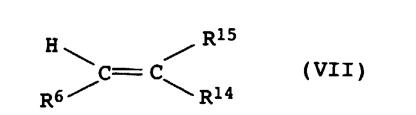 where
- the radical R⁶ is hydrogen or a carboxyl group or a functional derivative of a carboxyl group,
- the radical R¹⁴ is a carboxyl group or a functional derivative of a carboxyl group and
- the radical R¹⁵ is hydrogen or methyl.

7. A fertilizer mixture comprising
A. a mineral fertilizer and
B. 100 to 10000 ppm by weight, based on the mineral fertilizer, of a pyrazole derivative as set forth in claims 1 to 6.

## Revendications

1. Utilisation de dérivés du pyrazole répondant à la formule générale I 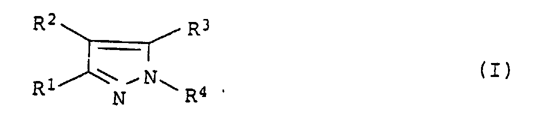 ou de leurs sels formés par addition avec des acides, en tant qu'inhibiteurs de la nitrification, les symboles R¹, R², R³ et R⁴ de la formule ayant les significations suivantes :
- R¹, R² et R³ représentent des halogènes, des groupes nitro, l'hydrogène, des groupes alkyle en C1-C20, cycloalkyle en C3-C8, aryle en C5-C20 ou alkylaryle, ces quatre substituants pouvant eux-mêmes porter un ou deux substituants halogéno et/ou hydroxy,
- R⁴ représente un groupe R^{4a} de formule II 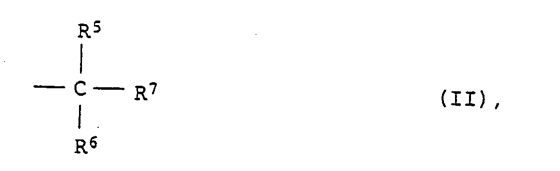 dans laquelle
- R⁵ et R⁶ représentent l'hydrogène, des groupes alkyle en C1-C20 qui peuvent eux-mêmes porter un ou deux substituants halogéno et/ou hydroxy, un groupe carboxyle, un groupe carboxyméthyle ou un dérivé fonctionnel des deux groupes mentionnés en dernier, et
- R⁷ représente un groupe carboxyle ou carboxy-(alkyle en C1-C3) ou un dérivé fonctionnel de ces groupes.

2. Utilisation de dérivés du pyrazole selon la revendication 1 en tant qu'inhibiteurs de nitrification, les symboles R¹, R² et R³ représentant des groupes alkyle en C1-C5 ou des halogènes.

3. Dérivés du pyrazole de formule Ia 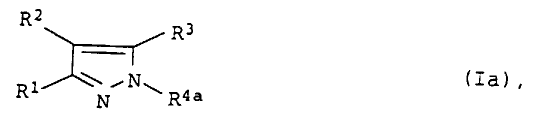 dans laquelle R² représente un halogène, ou leurs sels formés par addition avec des acides, à l'exception des composés pour lesquels deux des symboles R¹, R² ou R³ représentent simultanément des halogènes.

4. Dérivés du pyrazole selon la revendication 3 pour lesquels le groupe R^{4a} porte lui-même les substituants suivants :
- R⁵ : l'hydrogène,
- R⁶ : l'hydrogène ou un groupe carboxyle ou un dérivé fonctionnel du groupe carboxyle,
- R⁷ : un groupe carboxyméthyle, un groupe 1-carboxyéthyle ou un dérivé fonctionnel de ces groupes.

5. Procédé de préparation des dérivés du pyrazole selon la revendication 1, caractérisé par le fait que l'on fait réagir un dérivé du pyrazole répondant à la formule Va 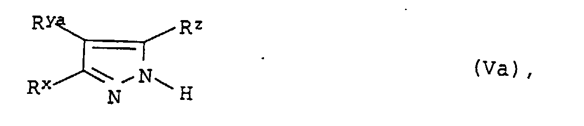 dans laquelle
- R^{x} et R^{z} représentent des halogènes, des groupes nitro, l'hydrogène, des groupes alkyle en C1-C20, cycloalkyle en C3-C8, aryle en C5-C20 ou alkylaryle, ces quatre derniers groupes pouvant eux-mêmes porter un ou deux substituants halogéno et/ou hydroxy, et
- R^{ya} représente un halogène,
avec un composé de formule VI 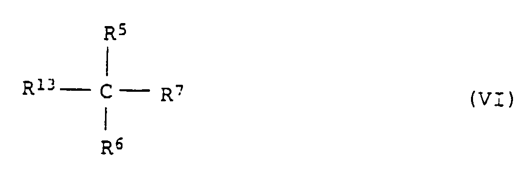 dans laquelle R¹³ représente le chlore ou le brome, à l'exception des composés pour lesquels deux des symboles R¹, R² ou R³ représentent simultanément des halogènes.

6. Procédé de préparation des dérivés du pyrazole selon la revendication 4, caractérisé par le fait que l'on fait réagir un dérivé du pyrazole de formule Va avec un composé de formule VII 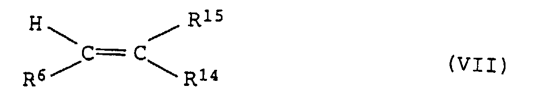 dans laquelle
- R⁶ représente l'hydrogène ou un groupe carboxyle ou un dérivé fonctionnel d'un groupe carboxyle,
- R¹⁴ représente un groupe carboxyle ou un dérivé fonctionnel d'un groupe carboxyle et
- R¹⁵ représente l'hydrogène ou un groupe méthyle.

7. Engrais mélangés contenant
A. un engrais minéral et
B. 100 à 10 000 ppm en poids, par rapport à l'engrais minéral, d'un dérivé du pyrazole selon les revendications 1 à 6.
